(19) Europäisches Patentamt / European Patent Office / Office européen des brevets

(11) **EP 4 360 540 A1**

(12) **DEMANDE DE BREVET EUROPEEN**

(43) Date de publication:
**01.05.2024   Bulletin 2024/18**

(21) Numéro de dépôt: **23201697.2**

(22) Date de dépôt: **04.10.2023**

(51) Classification Internationale des Brevets (IPC):
**A61B 5/00** *(2006.01)*    **A61B 5/0245** *(2006.01)*
**A61B 5/308** *(2021.01)*    **A61B 5/332** *(2021.01)*
**A61B 5/024** *(2006.01)*    **A61B 5/1455** *(2006.01)*

(52) Classification Coopérative des Brevets (CPC):
**A61B 5/681; A61B 5/02416; A61B 5/0245;
A61B 5/14552; A61B 5/308; A61B 5/332;
A61B 5/7225; A61B 5/742;** A61B 2562/0209

(84) Etats contractants désignés:
**AL AT BE BG CH CY CZ DE DK EE ES FI FR GB
GR HR HU IE IS IT LI LT LU LV MC ME MK MT NL
NO PL PT RO RS SE SI SK SM TR**
Etats d'extension désignés:
**BA**
Etats de validation désignés:
**KH MA MD TN**

(30) Priorité:  **24.10.2022   FR 2211022**

(71) Demandeur: **Withings
92130 Issy-Les-Moulineaux (FR)**

(72) Inventeurs:
• **Rafik, Walid
92370 Issy-les-Moulineaux (FR)**
• **Willemet, Marie
92370 Issy-les-Moulineaux (FR)**

(74) Mandataire: **Withings IP
2, rue Maurice Hartmann
92130 Issy-les-Moulineaux (FR)**

(54)   **MONTRE ECG CONNECTEE SIMPLIFIEE**

(57)      Il est proposé un dispositif électronique portatif configuré pour être positionné sur un poignet d'un utilisateur et permettant d'effectuer un électrocardiogramme (ECG). Le dispositif comprend exactement deux électrodes ECG, le potentiel étant imposé sur l'une des deux électrodes ECG. Les deux électrodes consistent en une première électrode ECG (220) sur le fond de carrure (210) et configurée pour être en contact avec la peau du poignet et une deuxième électrode ECG.

FIG. 7

**EP 4 360 540 A1**

## Description

### Domaine technique

La présente invention concerne le domaine des montres connectées et en particulier des montres connectées capables d'effectuer un électrocardiogramme (ECG ou EKG). Par montre, il est signifié un dispositif portable (« wearable » en anglais), dont la position préférentielle est le poignet (« wrist wearable » en anglais). La présente invention concerne aussi le domaine des montres connectées dites hybrides, c'est-à-dire des montres connectées présentant un aspect visuel plus proche des montres mécaniques conventionnelles, notamment grâce à la présence d'un train d'engrenages ainsi que d'aiguilles mécaniques pour indiquer au moins l'heure (aiguille des heures et aiguille des minutes).

### Technique antérieure

Un ECG est une mesure qui permet d'étudier le fonctionnement du coeur en mesurant son activité électrique. Une impulsion électrique traverse le coeur à chaque contraction et l'ECG constitue un tracé obtenu en enregistrant les potentiels électriques. L'ECG fait partie des mesures principales pour le suivi cardiovasculaire. Grâce à son intégration dans une montre, tout utilisateur peut régulièrement effectuer un ECG, ce qui permet une meilleure analyse et prévention des risques cardiaques. Pour effectuer un ECG, plusieurs électrodes sont positionnées sur le corps humain, afin de détecter différentes composantes des signaux électriques qui sont appelées dérivations ou voies (« leads » en anglais). Dans une des formes les plus simples, l'ECG permet d'obtenir une mesure bipolaire entre bras droit et bras gauche, appelée dérivation I ou DI.

Le test est indolore, passif et non invasif (pas d'injection de courant dans la peau) et peut être effectué en moins d'une minute. Bien que le concept théorique d'intégrer un ECG dans un dispositif grand public ait été présenté depuis plusieurs années (voir notamment le document US5289824), l'implémentation concrète et effective n'a été réussie que récemment. Il s'agit d'une innovation technologique majeure.

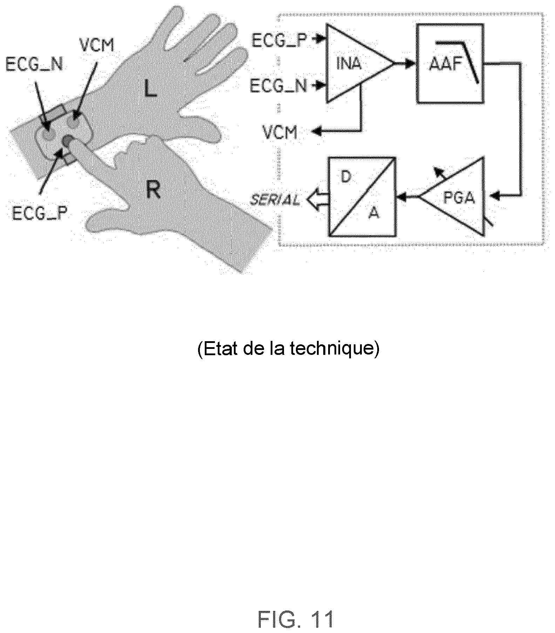

Le document « A Review of Methods for Non-Invasive Heart Rate Measurement on Wrist » (de Pinho Ferreira & AI, 2020) décrit l'état de l'art concernant les mesures cardiaques au poignet. Ce document explique que la mesure unipolaire de l'ECG à partir d'un seul endroit du corps humain ne serait pas possible en raison de sa nature différentielle, ainsi que du mode commun de ce signal qui est flottant et peut varier considérablement. Pour obtenir le ratio signal sur bruit (« Signal to Noise Ratio », SNR, en anglais) le plus élevé, deux électrodes sont généralement placées de chaque côté du coeur, là où se produisent les potentiels d'action cardiaques. Malheureusement, le signal est rapidement atténué lorsque les électrodes sont éloignées du coeur, et en particulier si les deux électrodes sont placées du même côté du coeur, comme par exemple le long d'un seul bras. Cependant, si la mesure est effectuée à partir des deux mains, l'amplitude reste suffisamment élevée pour extraire la fréquence cardiaque de manière pratique. Ainsi, l'approche conventionnelle consiste à placer un dispositif sur le poignet qui doit être touché par l'autre main, assurant ainsi le contact électrique avec la peau des deux bras, comme illustré sur la Figure 11 illustrant l'état de la technique : deux électrodes (ECG_N et VCM) sont placées sous le dispositif, au contact du poignet d'un bras (par exemple le bras gauche), et une électrode supérieure (ECG_P) permet de placer un doigt de la main opposée (par exemple celle du bras droit).

Les montres permettant d'effectuer un ECG (appelées « montre-ECG » par la suite) sont peu nombreuses sur le marché en 2022. On peut notamment citer la Withings Move ECG, la Withings ScanWatch, l'Apple Watch et la Samsung Galaxy.

Comme décrit ci-dessus, ces montres utilisent toutes trois électrodes ECG, dans une configuration similaire à celle de la figure 11 en ce qui concerne les contacts entre les bras et les électrodes. En particulier, la mesure d'ECG une-dérivation (lead I) consiste à mesurer la différence de potentiel électrique entre les membres supérieurs gauche et droit. Les trois électrodes se composent donc d'une électrode négative (ECG_N) au contact d'un bras, d'une électrode neutre au contact du même bras et d'une électrode positive (ECG_P) au contact de l'autre bras. Cet agencement permet un mouvement simple et répétable, en mettant une seule électrode au contact du bras qui doit se déplacer. Le rôle de l'électrode neutre est de fixer le corps à un mode commun (un potentiel moyen). Ceci permet de centrer le signal autour d'un potentiel moyen, et d'éliminer activement le bruit observé de façon commune sur les deux membres, à savoir principalement les interférences électromagnétiques dues aux sources de puissances électriques (bruit à 50 ou 60Hz).

L'implémentation d'un dispositif de mesure d'ECG dans une montre présente de nombreuses difficultés techniques, notamment en ce que les signaux électriques à identifier sont de faibles amplitudes et en ce que leur acquisition est fréquemment bruitée. Le bon positionnement des électrodes et la gestion de la chaîne électrique entre les électrodes et le module électronique qui gère l'ECG sont cruciaux.

### Exposé de l'invention

L'invention se propose de simplifier l'architecture d'une telle montre-ECG afin notamment de limiter les sources de bruit, de limiter les pièces mécaniques et/ou les étapes de fabrication, et de simplifier le montage électronique.

A cet effet, la présente description concerne un dispositif électronique portatif configuré pour être positionné sur un poignet d'un utilisateur, le dispositif portatif permettant d'effectuer un électrocardiogramme, ECG, ledit dispositif comprenant : un fond de carrure, configuré pour être au moins partiellement en contact avec la peau du poignet, le dispositif comprenant en outre exactement deux électrodes ECG, les deux électrodes ECG consistant en : une première électrode ECG, en matériau conducteur, sur le fond de carrure et configurée pour être en contact avec la peau du poignet, une deuxième électrode ECG, en matériau conducteur. Pour effectuer un électrocardiogramme, le dispositif comprend en outre un module électronique ECG, électriquement connecté à la première électrode ECG et à la deuxième électrode ECG, et configuré pour imposer un potentiel sur l'une des deux électrodes ECG et pour mesurer un potentiel de l'autre des deux électrodes ECG, dont le potentiel varie en fonction des battements du coeur de l'utilisateur La deuxième électrode ECG est agencée sur le dispositif électronique portatif de façon à pouvoir être en mise en contact avec le bras de l'utilisateur opposé à celui qui est en contact avec la première électrode ECG.

En effet, il est proposé un dispositif électronique portatif avec uniquement deux électrodes ECG Le dispositif électronique portatif mesure directement la différence de potentiel entre l'une des deux électrodes ECG, par exemple celle en contact avec le poignet, et un potentiel électrique imposé sur l'autre électrode, par exemple celle incorporée dans la lunette de la montre. Le dispositif électronique portatif permet de mesurer la différence de potentiel entre le potentiel variable à l'une des deux électrodes ECG, et un potentiel imposé à l'autre électrode, contrairement à une configuration trois électrodes où la différence de potentiel est calculée entre deux électrodes ECG à potentiel variable. Le potentiel imposé est typiquement constant, pour simplifier l'architecture électronique.

Cette configuration à deux électrodes ECG dans une montre n'a pas été proposée auparavant du fait de la nécessité de limiter les interférences électromagnétiques dues aux sources de puissances électriques. Toutefois, les montres modernes fonctionnent grâce à une alimentation réduite provenant d'une batterie interne à la montre. La connexion continue à un réseau d'alimentation électrique n'est alors plus nécessaire. Les inventeurs ont eu l'idée de proposer une architecture à deux électrodes, malgré les difficultés qu'une telle solution peut laisser présumer. En effet, outre la question des interférences, l'agencement des deux électrodes est nécessairement différent des architectures conventionnelles des montres à trois électrodes ECG. Ainsi, les inventeurs ont mis au point une architecture de montre ECG avec uniquement deux électrodes ECG et ils ont mené des campagnes de test. Ils se sont ainsi rendu compte que, pour autant que la mesure soit réalisée sans contact direct avec une source d'alimentation connectée au réseau, les interférences électromagnétiques sont minimes et ne nécessitent pas la présence de l'électrode neutre. Les inventeurs ont alors proposé cette nouvelle architecture simplifiée à deux électrodes ECG pour réaliser un ECG sur une montre.

Cette architecture présente de nombreux avantages. En effet, elle permet la simplification de l'architecture des pièces puisqu'une seule électrode est requise sur le fond de carrure, contre deux auparavant. Cette électrode est en conséquence optimisée car un agrandissement de la surface de celle-ci est rendu possible. En outre, l'invention permet d'utiliser moins de composants dans le circuit électronique tout en ne subissant pas de perte d'amplitude par l'injection d'un potentiel de mode commun via l'électrode à potentiel imposé.

Dans un mode de réalisation, le dispositif comprend en outre une carrure, une glace et une lunette montée sur la carrure et entourant la glace, dans lequel la lunette comprend un corps de lunette et le corps de lunette comprend la deuxième électrode ECG.

Dans un mode de réalisation, la lunette est mobile en rotation par rapport à la carrure.

Dans un mode de réalisation, la carrure comprend une paroi latérale, la paroi latérale comprenant une ouverture dans laquelle est insérée une couronne mobile en rotation et/ou être mobile en translation, la deuxième électrode ECG étant formée par la couronne.

Dans un mode de réalisation, le potentiel imposé est un potentiel constant. Cela permet de simplifier l'architecture électronique et le traitement des signaux électriques pour générer un ECG.

Dans un mode de réalisation, le module ECG est configuré pour imposer le potentiel sur la deuxième électrode ECG.

Dans un mode de réalisation, le module ECG est configuré pour imposer le potentiel sur l'électrode négative.

Dans un mode de réalisation, le module ECG comprend un frontal analogique propre à recevoir une amplitude maximale de tension, le module ECG étant configuré pour imposer le potentiel constant à une valeur environ égale à la moitié de l'amplitude maximale de tension.

Dans un mode de réalisation, le module ECG est configuré pour imposer le potentiel à une valeur comprise entre 0,5V et 2 V, notamment entre 0,8V et 1,0V. En particulier, le potentiel imposé est constant.

Dans un mode de réalisation, le fond de carrure comprend un capteur optique, la première électrode entourant au moins en partie le capteur optique.

Dans un mode de réalisation, la première électrode entoure le capteur optique sur une étendue angulaire supérieure à 180°.

Dans un mode de réalisation, la première électrode entoure entièrement le capteur optique.

Dans un mode de réalisation, la première électrode présente une forme annulaire.

Dans un mode de réalisation, la première électrode se présente sous la forme d'un corps métallique.

Dans un mode de réalisation, la première électrode se présente sous la forme d'un revêtement métallique.

Dans un mode de réalisation, le dispositif électronique portatif est une montre, la montre étant par exemple une montre hybride avec des aiguilles mécaniques.

La présente invention concerne aussi une méthode de prise d'un électrocardiogramme, ECG, à l'aide d'un dispositif tel que défini ci-dessus, durant laquelle l'utilisateur met un bras en contact avec la première électrode ECG et un autre bras en contact avec la deuxième électrode ECG.

La présente invention concerne aussi une utilisation d'un dispositif tel que précédemment décrit pour réaliser un électrocardiogramme.

**Brève description des dessins**

D'autres caractéristiques, détails et avantages apparaîtront à la lecture de la description détaillée ci-après, et à l'analyse des dessins annexés, sur lesquels :

**[Fig. 1]** Cette figure présente une vue du dessus (selon une direction Z) de la montre-ECG, où le cadran, la glace et la lunette en particulier sont visibles, selon un premier mode de réalisation (les aiguilles ne sont pas représentées).

**[Fig. 2]** Cette figure présente une vue du dessous (selon une direction Z) de la montre-ECG, où le fond de carrure et la carrure en particulier sont visibles, selon le premier mode de réalisation.

**[Fig. 3]** Cette figure présente une vue en coupe selon le plan XZ d'une montre-ECG, selon un mode de réalisation.

**[Fig. 4]** Cette figure présente un diagramme de la montre-ECG avec certains composants, notamment électroniques.

**[Fig. 5]** Cette figure illustre le fonctionnement de la montre-ECG.

**[Fig. 6]** Cette figure présente une vue du dessus (selon une direction Z) de la montre-ECG selon un deuxième mode de réalisation.

**[Fig. 7]** Cette figure présente une vue du dessous en perspective de la montre-ECG selon le deuxième mode de réalisation.

**[Fig. 8]** Cette figure présente une vue du dessous en perspective de montres-ECG selon d'autres modes de réalisation.

**[Fig. 9] [Fig. 10]** Ces figures présentent des résultats de tests comparatifs de la montre-ECG selon le deuxième mode de réalisation avec une montre ECG avec une configuration 3 électrodes ECG.

**[Fig. 11]** Cette figure illustre une montre ECG selon l'état de la technique.

**Description détaillée des modes de réalisation**

La présente description concerne un dispositif électronique portatif comprenant un capteur d'électrocardiogramme (ci-après : capteur ECG). Dans un mode de réalisation particulier, qui est celui illustré, le dispositif électronique portatif est une montre (ci-après : montre-ECG). La montre-ECG peut comprendre un bracelet. Néanmoins, dans le cadre de la présente description, le terme montre-ECG n'inclut pas nécessairement le bracelet, qui est généralement fabriqué ailleurs et qui peut être assemblé aux endroits de vente.

Le dispositif électronique portatif est connecté, de sorte qu'il peut échanger des données à distance (sans-fil) de façon bidirectionnelle avec un terminal, de type smartphone. La connexion peut se faire par Bluetooth, comme un Bluetooth Low Energy (BLE). En particulier, les données échangées de la montre-ECG vers le terminal sont des données d'ECG acquises par la montre-ECG. La montre-ECG peut aussi recevoir des données par le terminal (données d'heure, d'alarme, notifications, etc.).

Dans un mode de réalisation, la montre-ECG est une montre hybride, c'est-à-dire une montre avec un cadran et des aiguilles pour indiquer les heures et les minutes.

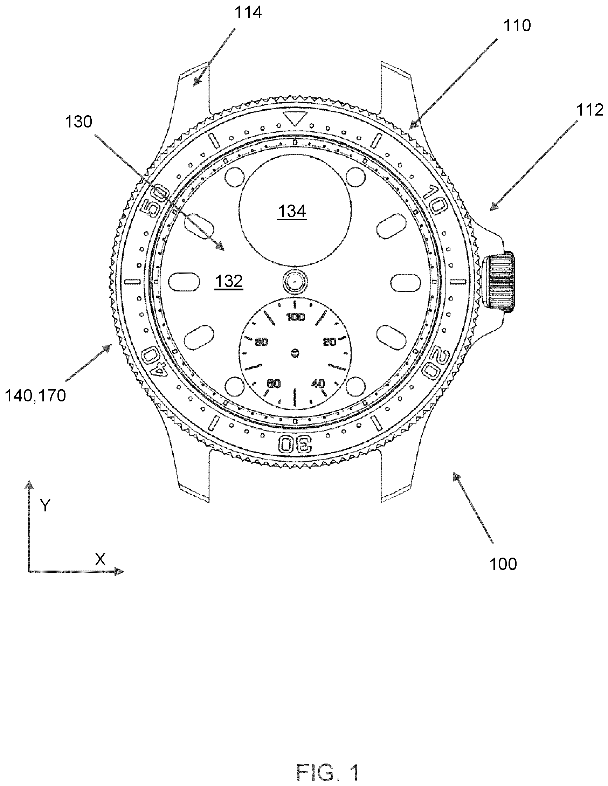

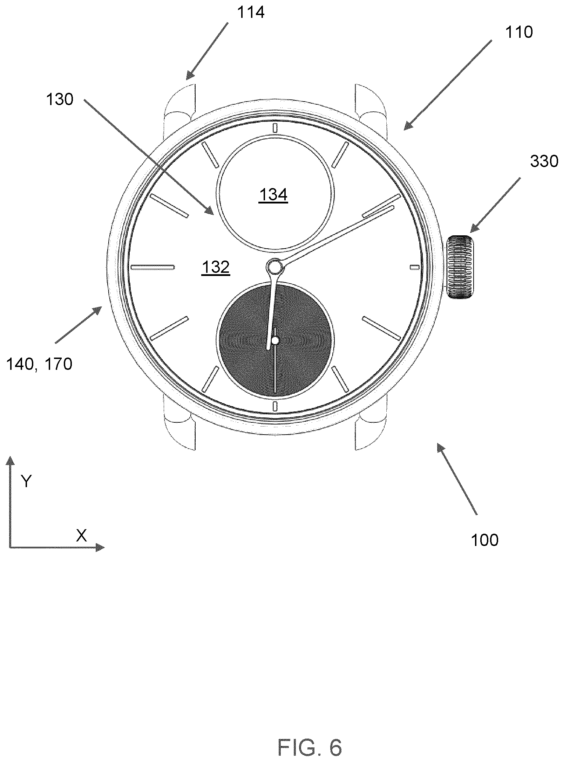

Les figures 1 à 3 représentent une montre-ECG 100 électronique, de type hybride (avec un cadran, des aiguilles mécaniques, et éventuellement un écran intégré au cadran) selon un premier exemple et les figures 6 à 7 représentent une montre-ECG 100 électronique selon un deuxième exemple.

Dans la présente description, la notion de « dessus », « dessous » est définie par rapport à la direction Z, le dessus étant en direction de la glace et le dessous étant en direction du fond de carrure, qui vont être décrits ci-après.

La montre-ECG 100 peut comprendre une carrure 110 et un fond de carrure 210 qui est configuré pour être au moins partiellement au contact de la peau du poignet de l'utilisateur. La carrure 110 et le fond de carrure 210 sont solidaires entre eux. Dans un mode de réalisation, comme illustré sur les figures, la carrure 110 et le fond de carrure 210 sont deux pièces distinctes. Dans un mode de réalisation non illustré sur les figures, le fond de carrure 210 est une pièce intégrée à la carrure 110. La carrure 110 peut comprendre une paroi latérale 112, qui est généralement visible lorsque la montre-ECG 100 est portée au poignet. La carrure 110 peut comprendre des cornes 114 (deux paires, de part et d'autre de la carrure 110) pour attacher un bracelet (non représenté sur les figures). La carrure 110 peut inclure une pluralité de pièces.

La montre-ECG 100 peut aussi comprendre une glace 130 (« *glass* » ou « *crystal* » en anglais), généralement montée sur la carrure 110, de sorte que la glace 130 soit fixe. La glace 130 est ou peut comprendre un verre protecteur typiquement transparent et peut être réalisé en verre, en céramique, en plastique ou n'importe quel matériau transparent. Le contour de la glace 130 est ici typiquement de forme circulaire.

Dans le cas d'une montre hybride, sous la glace 130, la montre-ECG 100 comprend en outre un cadran 132 avec des aiguilles (des aiguilles physiques, qui ne sont pas représentées sur les figures). Le cadran 132 peut en outre recevoir un écran 134 (par exemple avec une ouverture dans le cadran qui permet de rendre visible un écran positionné juste sous le cadran), qui occupe par exemple un faible espace sous ou dans le cadran 132. Le glace 130 protège ces pièces et permet de les voir par transparence.

Dans le cas d'une « *smartwatch* » type Apple Watch, sous la glace 130, la montre-ECG comprend un écran, non représenté ici, qui occupe une largeur proche de la largeur de la montre-ECG 100. Dans un mode de réalisation, l'écran peut afficher des aiguilles. La glace 130 est alors le verre protecteur de l'écran.

La montre-ECG 100 peut comprendre en outre une lunette 140 (« *bezel* » en anglais), montée sur la carrure 110. La lunette 140 est positionnée autour de la glace 130 (radialement externe à la glace). La lunette 140 est une pièce de forme annulaire, essentiellement de révolution autour de la direction Z (à quelques modifications près).

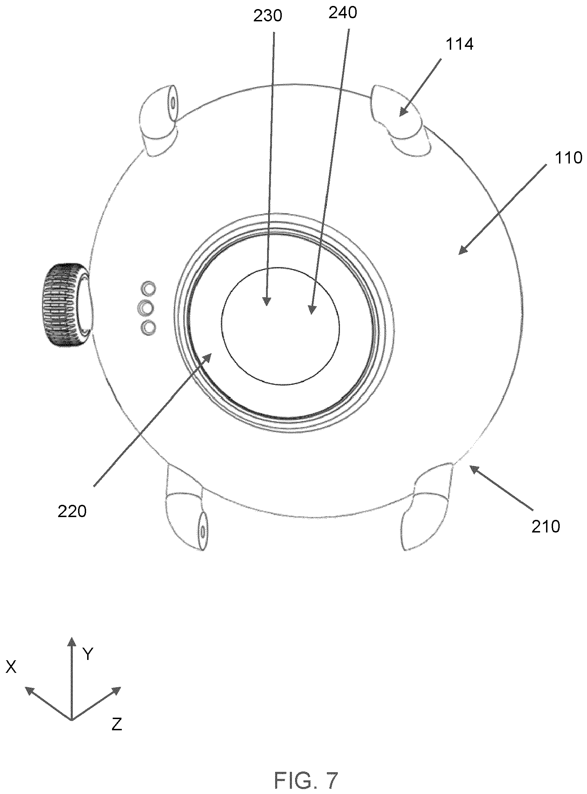

Dans l'exemple illustré sur les figures 1 à 3, la lunette 140 est montée à rotation par rapport à la carrure 110. La rotation peut se faire pas à pas (par exemple cran à cran). L'utilisateur peut ainsi faire tourner la lunette 140. En variante, dans l'exemple illustré sur les figures 6 et 7, la lunette 140 est fixe par rapport à la carrure 110. Ainsi dans cette variante, la lunette 140 n'est pas rotative par rapport à la carrure 110.

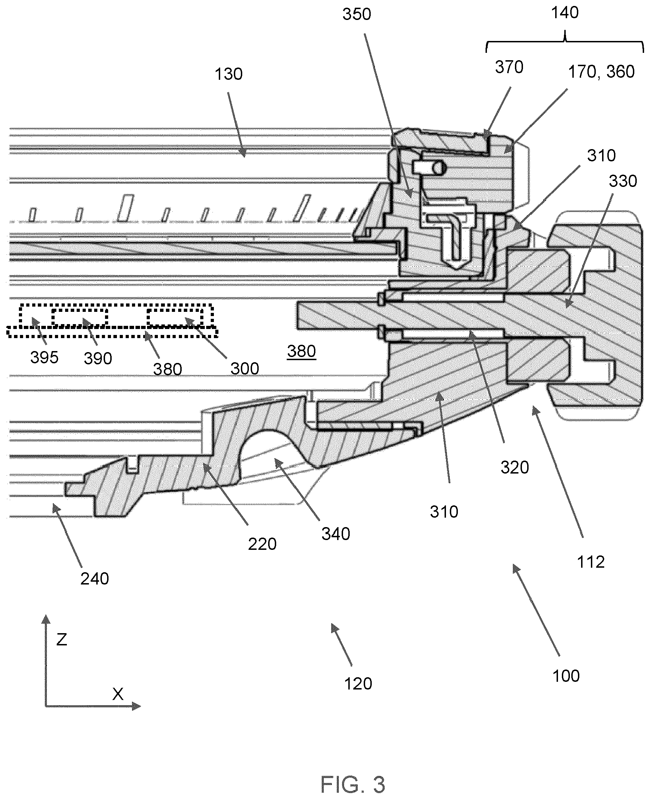

La figure 3 illustre une vue en coupe de la montre-ECG 100 selon le premier exemple. La carrure 110 comprend notamment un corps principal 310 (ou corps de carrure), qui comprend la paroi latérale 112. Sur la figure 3, le corps principal 310 comprend une ouverture 320 au niveau de la paroi latérale 112 pour permettre la mise en place de la couronne 330. La couronne 330 présente notamment un rôle d'interface utilisateur entre la montre-ECG 100 et l'utilisateur. A cette fin, la couronne 330 peut être mobile en rotation (ici autour de l'axe X) et/ou être mobile en translation (ici le long de l'axe X).

Le fond de carrure 210 peut être monté sur le corps principal 310. Dans la vue en coupe de la figure 3, le fond de carrure 210 comprend un renfoncement 340, qui est un plot de positionnement pour une station de charge de batterie. La carrure 110 peut aussi comprendre un support de lunette 350, monté sur le corps principal 310. La lunette 140 est notamment montée sur le support de lunette 350, dans une position radialement externe. La glace 130 est elle aussi montée sur le support de lunette 350, dans une position radialement interne.

La lunette 140 peut comprendre un corps de lunette 360, qui est monté sur la carrure 110. Le corps de lunette 360 peut intégrer directement (par gravure ou autre) le décor traditionnel d'une montre, de sorte que la lunette 140 soit formée intégralement par le corps de lunette 360. Alternativement, comme illustré en figure 3, la lunette 140 comprend en outre un anneau de visualisation 370 (aussi appelé « décor ») qui peut être monté (par exemple par collage, par exemple avec de l'adhésif double face) sur une face supérieure du corps de lunette 360. Le décor peut alors comprendre des marqueurs visuels.

Le corps de lunette 360 peut être en matériau conducteur, comme du métal (par exemple en acier inoxydable ou en alliage de titane). Alternativement, le corps de lunette 360 peut être en plastique chargé ou en céramiques conductrices. Alternativement encore, le corps de lunette 360 peut ne pas être conducteur et un revêtement conducteur est déposé sur tout ou partie du corps de lunette 360 (par exemple de la face supérieure ou de la face latérale externe jusqu'aux crans de la face inférieure).

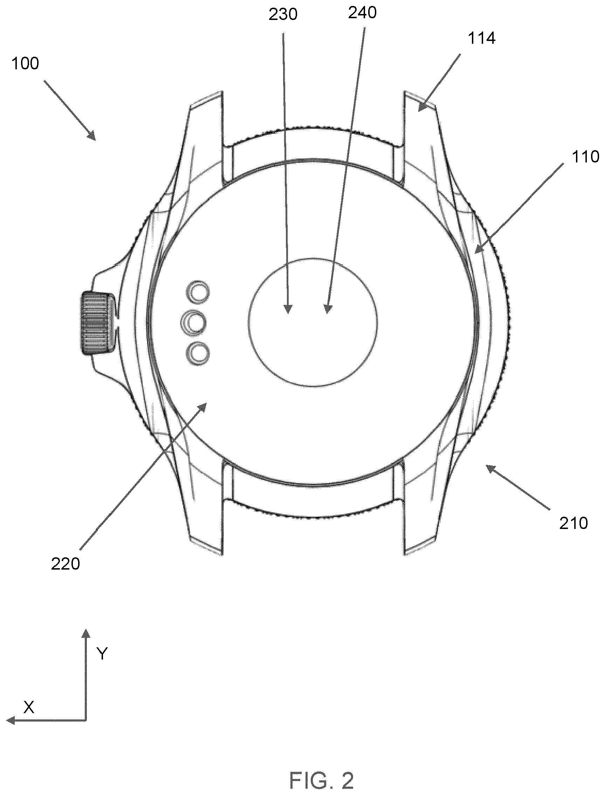

La montre-ECG 100 comprend un capteur optique 230, visible en figure 2. Le capteur optique 230 comprend au moins une source de lumière (par exemple une LED), pour émettre de la lumière, et au moins une photodiode, pour recevoir de la lumière. Le capteur optique 230 est typiquement un capteur PPG (photopléthysmographie). Le capteur optique 230 peut être positionné derrière une lentille 240, par exemple en verre, qui vient à l'interface de la peau du poignet. Le document PCT/EP2021/058955, au nom de Withings décrit en détail le capteur optique, qui se retrouve sur la Withings ScanWatch.

Pour récupérer les signaux électriques générés par le corps humain, la montre-ECG 100 comprend un capteur ECG. Le capteur ECG comprend notamment un ensemble d'électrodes (appelées électrodes ECG et décrites en détail par la suite) et un module électronique ECG 300 (illustré schématiquement sur la figure 3), auquel les électrodes ECG sont électriquement connectées.

Par électrode, il est signifié une pièce conductrice apte à recevoir ou transmettre un courant électrique, un potentiel électrique ou une tension électrique. La pièce peut être en matériau conducteur ou comprendre un revêtement conducteur. Par « conducteur », il est signifié « conducteur d'électricité ».

Selon la présente description, la montre-ECG 100 comprend exactement deux électrodes ECG permettant d'effectuer un ECG, dont une première électrode ECG et une deuxième électrode ECG. Ainsi, contrairement aux montres-ECG conventionnelles qui nécessitent trois électrodes ECG afin de réaliser un ECG, la présente description propose de simplifier l'architecture de la montre-ECG 100, comme cela sera expliqué plus en détail par la suite.

Comme visible sur les figures 2 et 3, la première électrode ECG 220 se trouve sur le fond de carrure 210 afin d'être en contact avec la peau du poignet de l'utilisateur sur lequel ce dernier porte la montre-ECG 110. La première électrode 220 est électriquement connectée au module ECG 300. La première électrode ECG 220 peut entourer au moins en partie le capteur optique 230.

La première électrode 220 est réalisée en matériau conducteur, comme du métal (par exemple en acier inoxydable ou en alliage de titane). Dans l'exemple illustré sur les figures 1 à 3, la première électrode 220 se présente sous la forme d'un corps métallique. Par corps métallique, on entend une pièce dont l'épaisseur maximale est supérieure à 0,1 mm.

Dans la variante illustrée par les figures 6 et 7, la première électrode 220 peut être formée par un revêtement conducteur déposé sur une surface (qui est elle-même conductrice ou non-conductrice). En particulier, la première électrode 220 se présente sous la forme d'un revêtement métallique sur la lentille 240 où se trouve le capteur optique 230.

Dans les deux exemples illustrés sur les figures 1 à 7, la première électrode 220 présente une forme annulaire.

Dans les exemples illustrés, la première électrode 220 entoure au moins en partie le capteur optique 230. En particulier, la première électrode 220 peut entourer complètement le capteur optique 230 comme visible sur les figures 2 et 7.

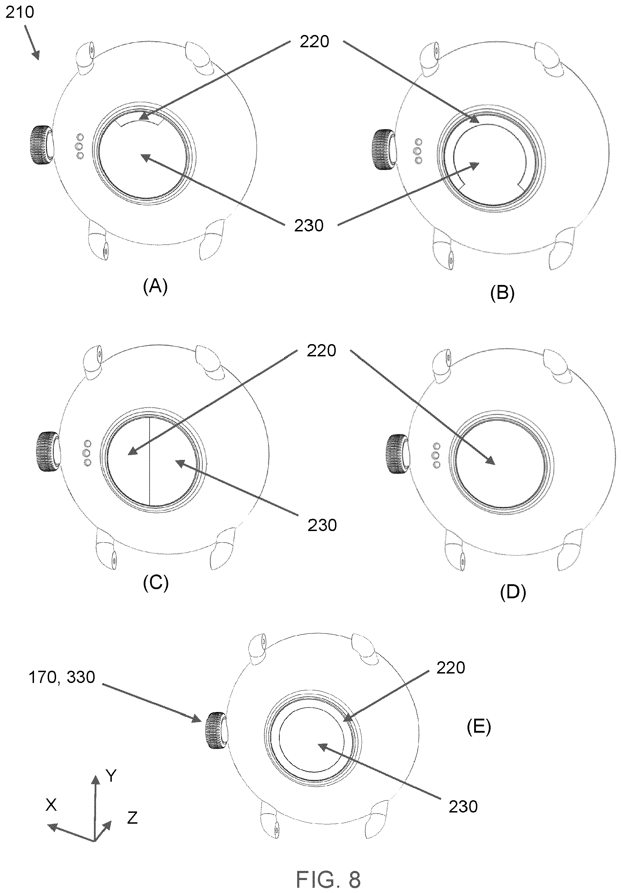

La figure 8 illustre d'autres exemples d'agencements pour la première électrode 220.

Dans l'exemple (A) de la figure 8, la première électrode 220 peut s'étendre autour du capteur optique 230 sur une étendue angulaire inférieure à 180°, par exemple 90°. La première électrode 220 est représentée en haut selon la direction Y, mais peut se situer en variante n'importe où autour du capteur optique 230.

Dans l'exemple (B) de la figure 8, la première électrode 220 peut s'étendre autour du capteur optique 230 sur une étendue angulaire supérieure à 180°, mais inférieure à 360°, par exemple environ 270°. La première électrode 220 est représentée en haut selon la direction Y, mais peut se situer en variante n'importe où autour du capteur optique 230.

Dans l'exemple (C) de la figure 8, la première électrode 220 et le capteur optique 230 peuvent être formés par deux demi-disques se faisant face. La première électrode 220 est représentée à gauche selon la direction X, mais peut se situer en variante à droite selon la direction X.

Dans l'exemple (D) de la figure 8, la montre-ECG 100 ne comprend pas de capteur optique 230 et la première électrode 220 se présente sous la forme d'un disque disposé sensiblement au centre du fond de carrure 210.

La deuxième électrode 170 est elle-aussi électriquement connectée au module ECG 300.

Comme visible sur les figures 1 et 3, la deuxième électrode ECG 170 peut se trouver sur la lunette 140 afin de pouvoir être facilement en contact avec la peau de l'autre main de l'utilisateur. Comme illustré sur les figures, la deuxième électrode 170 est le corps de lunette 360, c'est-à-dire que l'intégralité de la lunette 140 ou une partie de la lunette (accessible par l'utilisateur) forme l'électrode 170. L'utilisateur peut ainsi toucher la lunette en n'importe quel endroit. Comparativement à une couronne faisant office d'électrode, la montre-ECG 100 peut être portée à la main gauche et la main droite indifféremment, sans que les gestes à effectuer pour effectuer un ECG soient les mêmes pour un utilisateur portant la montre à gauche ou à droite (symétrie d'utilisation), à l'inverse des couronnes-électrodes décrites en introduction.

En variante, comme illustré sur l'exemple (E) de la figure 8, la deuxième électrode ECG 170 est formée par la couronne 330. Afin d'effectuer une mesure ECG, l'utilisateur place alors sa main libre en contact avec la couronne 330.

Alternativement, dans un exemple non illustré, la deuxième électrode se trouve ou fait partie d'un bouton présent dans la carrure.

La deuxième électrode 170 est réalisée en matériau conducteur, comme du métal (par exemple en acier inoxydable ou en alliage de titane). Dans les exemples illustrés, la deuxième électrode 170 se présente sous la forme d'un corps métallique.

La carrure 110 (et en particulier le fond de carrure 210, le cadran 132 et le corps principal 310 de la carrure 110) définit un volume interne 380 apte à recevoir différents composants, comme des composants électroniques. Ces composants électroniques sont ainsi protégés de l'eau ou de la poussière (avec les étanchéités appropriées, notamment permises par les joints précités).

Le module ECG 300 est représenté schématiquement en pointillé sur la figure 3 et positionné schématiquement. Les deux électrodes ECG 220, 170 sont électriquement connectées au module ECG 300. Le module ECG 300 est configuré pour récupérer des signaux électriques venant du corps humain et pour, après traitement, générer un électrocardiogramme. Le module ECG 300 peut être monté sur une carte électronique 380 (représenté schématiquement en pointillé sur la figure 3 et positionné schématiquement), de type circuit imprimé (« *printed circuit board* », PCB en anglais), où d'autres éléments de la montre-ECG sont aussi montés.

Le module ECG 300 comprend notamment un frontal analogique (AFE pour *« Analog front-end »*, en anglais). Le frontal analogique est configuré pour recevoir au niveau de deux entrées (une entrée positive et une entrée négative) les signaux issus respectivement des deux électrodes ECG 220, 170 (qui sont ainsi définies comme une électrode positive et une électrode négative) et fournir en sortie un signal ECG. Le frontal analogique est par exemple le AD8233 proposé par Analog Devices. Le frontal analogique accepte une amplitude maximale de tension, correspondant à la tension d'alimentation du frontal analogique. Typiquement, cette amplitude maximale peut être comprise entre 1V et 4V, par exemple environ 1,8V.

Le module ECG 300 est configuré pour imposer un potentiel sur l'une des deux électrodes ECG. Le potentiel est notamment imposé par le frontal analogique de manière passive. Le potentiel imposé est avantageusement un potentiel constant. Dans un mode de réalisation, le potentiel constant est égal à environ la moitié de l'amplitude maximale de tension du frontal analogique. Par environ, il est entendu plus ou moins 10% de la valeur. En particulier, le potentiel imposé est compris entre 0,5V et 2V, par exemple entre 0,8V et 1,0V, notamment 0,9V.

En effet, si un potentiel n'était pas imposé sur l'une des deux électrodes ECG, les deux électrodes ECG seraient laissées "flottantes", c'est à dire qu'elles ne seraient pas reliées à une valeur de référence. Il existerait bien une différence de potentiel d'amplitude entre les 2 électrodes, mais les potentiels individuels pourraient prendre une multitude de valeurs. Or, comme expliqué précédemment, le frontal analogique accepte une amplitude de tension limitée. Dans l'exemple dans lequel cette amplitude maximale est environ égale à 1,8V, imposer le potentiel constant à environ 0,9V sur l'une des deux électrodes permet de ramener la mesure dans l'amplitude acceptable pour le module ECG 300. La mesure va alors osciller autour de 0,9V, et sera mesurable par le frontal analogique. Ainsi, les inventeurs ont déterminé qu'en se plaçant à la moitié de l'amplitude maximale, l'acquisition de déviations est optimisée vers le haut ou le bas.

Avantageusement, le module ECG 300 impose le potentiel sur la deuxième électrode ECG 170, qui est configurée pour être en contact avec le bras opposé à celui qui porte le dispositif ECG 100. Ainsi, contrairement à une configuration conventionnelle à 3 électrodes ECG où l'électrode de référence est placée sur le fond de carrure, le module ECG 300 impose le potentiel sur l'électrode ECG mise en contact avec la main libre (c'est-à-dire la main liée au poignet où ne se trouve pas la montre). En particulier, le module ECG 300 est configuré pour imposer le potentiel sur l'électrode négative, qui est notamment la deuxième électrode ECG 170. Les inventeurs se sont rendu compte qu'imposer le potentiel sur l'électrode en contact avec la main libre permet de réduire les fluctuations de potentiels induites par le contact de cette main libre avec la lunette et de réduire les micro-variations induites par le système nerveux (à l'inverse du contact entre la première électrode 220 sur le fond de carrure et le bras sur lequel est monté le dispositif ECG 100, notamment parce que le bras demeure immobile et inactif pendant la mesure).

Le potentiel à l'autre électrode est laissé libre par le module ECG 300. De la sorte, le potentiel de cette électrode correspond au potentiel du corps de l'utilisateur (lorsqu'il y a contact) et varie en fonction notamment des battements du coeur de ce dernier. Avantageusement, le module ECG 300 laisse le potentiel de la première électrode 220 libre.

Le capteur optique 230 est connecté à un module PPG 390, lui-aussi positionné dans le volume interne, qui peut être lui-aussi monté sur la carte électronique 380 de la montre-ECG 100. Le module PPG 390 est configuré pour générer les consignes à destination des sources de lumières du capteur optique 230 et pour récupérer les signaux électriques issus des photodiodes.

Une unité de contrôle 395 permet de piloter l'électronique embarqué sur la montre-ECG 100. L'unité de contrôle 395 peut par exemple inclure ou partiellement inclure le module ECG et le module PPG.

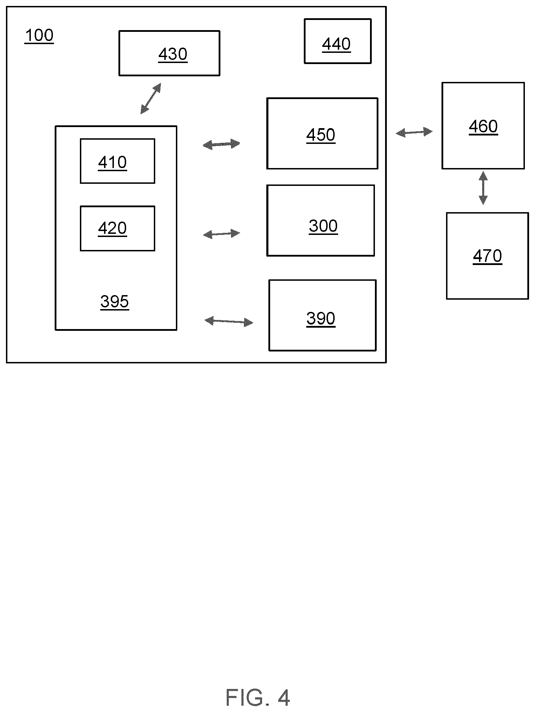

Comme représenté en figure 4 qui illustre schématiquement le dispositif 100, l'unité de contrôle 395 peut comprendre un ou des processeur(s) 410 et une mémoire 420 qui stocke des instructions aptes à être exécutées par le processeur 2302.

La montre-ECG 100 peut également comprendre un accéléromètre 430, relié à l'unité de contrôle 395 (pour le suivi de sommeil, d'activité, etc.).

Pour alimenter les différents composants en énergie électrique, la montre-ECG 100 comprend une batterie 440, par exemple une pile ou une batterie rechargeable. La batterie 440 est configurée pour alimenter notamment le module ECG 300. Le renfoncement 340 décrit précédemment permet de placer le fond de carrure 210 sur une station de charge pour recharger la batterie 440.

La montre-ECG 100 comprend un module de communication sans fil 450, tel qu'un module Bluetooth ou Bluetooth Low Energy ou un module Wi-Fi ou un module cellulaire (GSM, 2G, 3G,4G, 5G, Sigfox, etc.), qui lui permet de communiquer de façon bidirectionnelle avec au moins un terminal externe 460, tel qu'un téléphone portable. Le terminal externe 460 peut ensuite communiquer (bidirectionnellement) avec un serveur distant 470 pour le stockage et le traitement de données. Alternativement ou complémentairement, le module de communication sans fil 440 peut communiquer directement avec le serveur distant 470, par exemple via le réseau cellulaire ou via un réseau Wi-Fi. Les données obtenues par la montre-ECG 100, comme un électrocardiogramme, mais aussi les indications de rythme cardiaque, d'activité ou de saturation en oxygène, sont transmises au terminal externe 460 via le module de communication sans fil 440. L'unité de contrôle 395 peut traiter certains signaux avant de les envoyer, pour limiter la taille des données.

Le module PPG 390 et le module ECG 300 sont eux-aussi connectés à l'unité de contrôle 395 ou sont intégrés et/ou partiellement intégrés à celle-ci. Le module ECG 300 est connu en soi et ne sera pas décrit en détail. Différents types de composants électroniques peuvent être compris dans le module ECG 300 (processeur, résistance, condensateur, etc.).

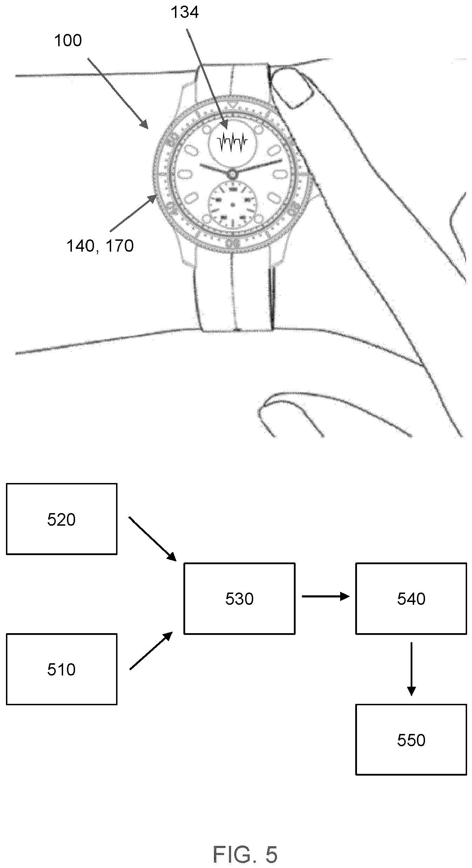

Le fonctionnement de la montre-ECG 100 va être expliqué par la suite, en référence à la Figure 5.

La montre-ECG 100 est initialement au poignet d'un utilisateur. L'utilisateur sélectionne alors le programme de mesure ECG, par exemple via la couronne 330 et l'écran 134.

Puis, l'utilisateur place au moins un doigt comme illustré en Figure 5, ou la paume de sa main, sur la deuxième électrode ECG 170 formée sur l'exemple illustré par la lunette 140. La mesure de l'ECG est ainsi effectuée de manière très simple pour l'utilisateur qui n'a qu'à poser sa main sur la lunette 140.

Un potentiel est imposé sur l'une des deux électrodes ECG lors d'une étape 510. Avantageusement, le potentiel est imposé par le module ECG 300 sur la deuxième électrode ECG 170, qui est l'électrode ECG mise en contact avec la main libre (c'est-à-dire la main liée au poignet où ne se trouve pas la montre, c'est-à-dire la main qui peut potentiellement bouger pendant la mesure et perturber les signaux). En imposant le potentiel sur cette main, la qualité de l'ECG est améliorée.

Le potentiel à l'autre électrode est laissé libre par le module ECG 300. De la sorte, le potentiel de cette électrode correspond au potentiel du corps de l'utilisateur (lorsqu'il y a contact) et varie en fonction notamment des battements du coeur de ce dernier. Dans une étape 520, le potentiel de la première électrode 220 en contact avec le poignet de l'utilisateur est mesuré. Ce poignet est généralement très immobile et non sollicité musculairement pendant la mesure de l'ECG. Par conséquent, les signaux obtenus par la première électrode 220 peuvent être de meilleure qualité.

Les étapes 510 et 520 peuvent être mises en oeuvre en parallèle.

Puis, lors de l'étape 530, le module ECG 300 mesure la différence de potentiels entre la première électrode 220 en contact avec le poignet et le potentiel électrique imposé sur la deuxième électrode 170 incorporée dans la lunette 140.

Puis, de manière connue en soi, la différence de potentiels est amplifiée, filtrée, numérisée et analysée par le module ECG 300 lors d'une étape 540.

Enfin, lors d'une étape 550, le résultat est par exemple affiché sur l'écran 134. En variante ou en complément, le résultat est envoyé au terminal externe 460 et/ou au serveur distant 470. Une notification peut alors être envoyé à l'utilisateur, sur l'écran 134 par exemple ou sur le terminal externe 460.

**Essais comparatifs**

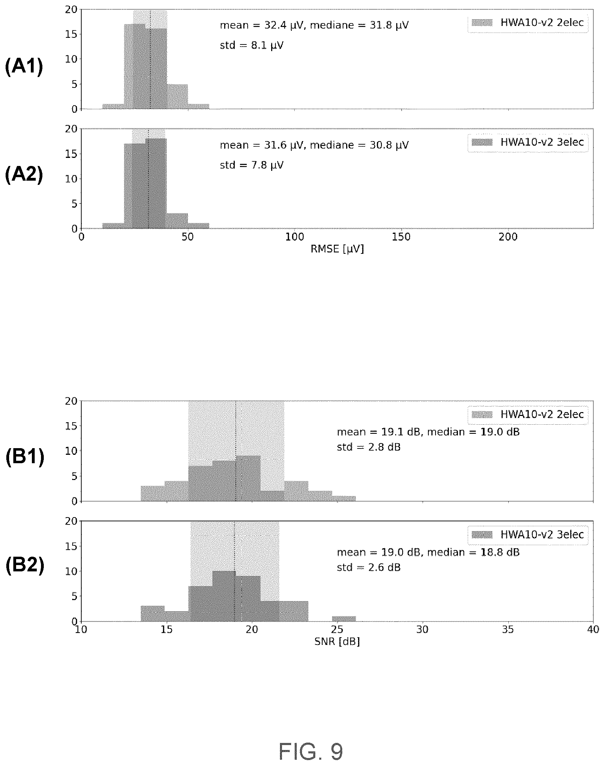

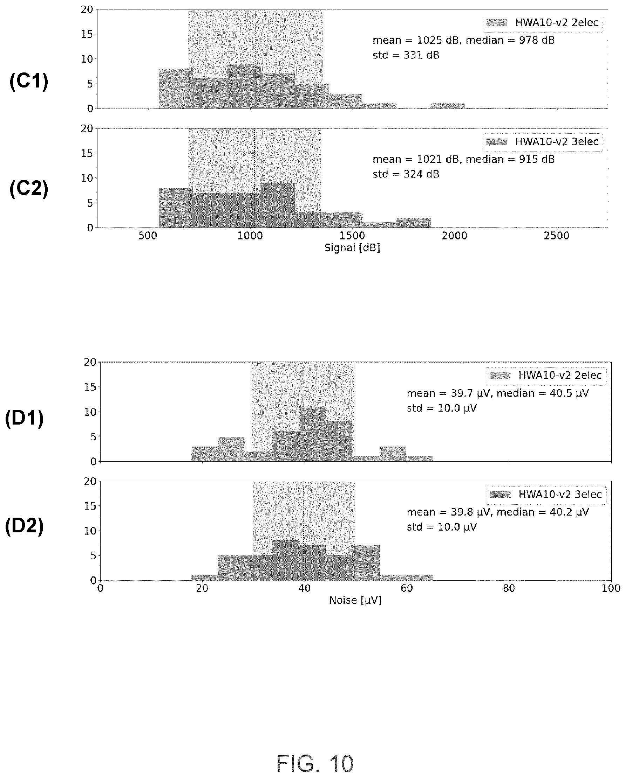

Les figures 9 et 10 illustrent des résultats de performance de la montre-ECG 100 illustrée sur les figures 6 et 7. Toutefois, l'homme du métier comprendra que ces résultats s'appliquent de manière similaire à la montre-ECG 100 illustrée sur les figures 1 à 3.

Les signaux ECG enregistrés par la montre-ECG 100 selon l'invention avec la nouvelle configuration 2-électrodes ECG sont comparés aux signaux ECG mesurés par une montre avec une configuration 3-électrodes classique. Les prototypes de montres utilisées présentent une mécanique, une architecture et des composants électroniques identiques. Seule la configuration des électrodes ECG varie. De plus, chaque mesure par un prototype de montre a été réalisée simultanément à une mesure ECG de référence avec un appareil de référence (Schiller).

Sur le graphe (A1) de la figure 9, l'ordonnée représente le nombre d'enregistrements d'ECG (40 au total) fait avec une montre et l'abscisse représente l'erreur quadratique moyenne (« *root mean squared error* », RMSE, en anglais) d'une montre-ECG 100 conforme au deuxième mode de réalisation par rapport à un électrocardiogramme de référence (Schiller Cardiovit FT-1). « *Mean* » signifie la moyenne et « *std* » signifie la déviation standard.

Le RMSE est calculé par rapport au signal de référence Schiller :

$$RMSE = \sqrt{\frac{\sum_{i=0}^{N} e_i^2}{N}}$$

avec N le nombre de points dans le signal et ei l'erreur au point i, correspondant à la différence entre le signal de référence Schiller et le signal de la montre testé au point i.

Sur le graphe (A2) de la figure 9, l'ordonnée représente le nombre d'enregistrements d'ECG (40 au total) fait avec une montre et l'abscisse représente l'erreur quadratique moyenne (« *root mean squared error* », RMSE, en anglais) d'une montre présentant une configuration 3 électrodes par rapport à un électrocardiogramme de référence (Schiller Cardiovit FT-1).

Sur le graphe (B1) de la figure 9, l'ordonnée représente le nombre d'enregistrements d'ECG (40 au total) fait avec une montre et l'abscisse représente le ratio signal sur bruit (« Signal to Noise Ratio », SNR, en anglais) d'une montre-ECG 100 conforme au deuxième mode de réalisation par rapport à un électrocardiogramme de référence (Schiller Cardiovit FT-1).

Le rapport Signal-sur-Bruit quantifie le niveau de bruit dans le signal ECG. Il est exprimé en décibels (dB) et se calcule de la manière suivante : SNR = 10 ∗ log(S/N), avec S la puissance du signal (S=s$^2$ /8) et N la puissance du bruit (N=n$^2$).

Dans cette métrique, le "signal s" est calculé comme l'amplitude du complexe QRS (sur une fenêtre de 100ms), et le "bruit n" comme la déviation standard de la partie supposée plate du signal ECG (une fenêtre de 200ms, qui commence 250 ms avant chaque complexe QRS détecté).

Sur le graphe (B2) de la figure 9, l'ordonnée représente le nombre d'enregistrements d'ECG (40 au total) fait avec une montre et l'abscisse représente le ratio signal sur bruit (« Signal to Noise Ratio », SNR, en anglais) d'une montre présentant une configuration 3 électrodes par rapport à un électrocardiogramme de référence (Schiller Cardiovit FT-1).

Sur le graphe (C1) de la figure 10, l'ordonnée représente le nombre d'enregistrements d'ECG (40 au total) fait avec une montre et l'abscisse représente l'amplitude du signal ECG d'une montre-ECG 100 conforme au deuxième mode de réalisation par rapport à un électrocardiogramme de référence (Schiller Cardiovit FT-1).

Sur le graphe (C2) de la figure 10, l'ordonnée représente le nombre d'enregistrements d'ECG (40 au total) fait avec une montre et l'abscisse représente l'amplitude du signal ECG d'une montre présentant une configuration 3 électrodes par rapport à un électrocardiogramme de référence (Schiller Cardiovit FT-1).

Sur le graphe (D1) de la figure 10, l'ordonnée représente le nombre d'enregistrements d'ECG (40 au total) fait avec une montre et l'abscisse représente l'amplitude du bruit d'une montre-ECG 100 conforme au deuxième mode de réalisation par rapport à un électrocardiogramme de référence (Schiller Cardiovit FT-1).

Sur le graphe (D2) de la figure 10, l'ordonnée représente le nombre d'enregistrements d'ECG (40 au total) fait avec une montre et l'abscisse représente l'amplitude du bruit d'une montre présentant une configuration 3 électrodes par rapport à un électrocardiogramme de référence (Schiller Cardiovit FT-1).

En référence à l'ensemble des métriques calculées et représentées sur les figures 9 et 10, on observe que les résultats obtenus par la montre-ECG 100 ne sont pas moins bons que ceux obtenus avec une montre avec une configuration 3 électrodes. Une telle montre à 3 électrodes ECG est notamment une ScanWatch 38mm, qui, en 2019, a obtenu la certification CE pour la mesure ECG.

## Revendications

1. Dispositif électronique portatif (100) configuré pour être positionné sur un poignet d'un utilisateur, le dispositif portatif (100) permettant d'effectuer un électrocardiogramme, ECG, ledit dispositif comprenant :

   - un fond de carrure (210), configuré pour être au moins partiellement en contact avec la peau du poignet,
   - exactement deux électrodes ECG, les deux électrodes ECG consistant en :

     ◦ une première électrode ECG (220), en matériau conducteur, sur le fond de carrure (210) et configurée pour être en contact avec la peau du poignet,
     ◦ une deuxième électrode ECG (170), en matériau conducteur,

   - un module électronique ECG (300), électriquement connecté à la première électrode ECG (220) et à la deuxième électrode ECG (170), et configuré pour imposer un potentiel sur l'une des deux électrodes ECG (220, 170) et pour mesurer un potentiel de l'autre des deux électrodes ECG (140, 170), afin d'effectuer un électrocardiogramme.

2. Dispositif selon la revendication 1, comprenant en outre une carrure (110), une glace (130) et une lunette (140) montée sur la carrure (110) et entourant la glace (130), dans lequel la lunette (140) comprend un corps de lunette (360) et le corps de lunette (360) comprend la deuxième électrode ECG (170).

3. Dispositif (100) selon la revendication 1, dans lequel la carrure (110) comprend une paroi latérale (112), la paroi latérale (112) comprenant une ouverture (320) dans laquelle est insérée une couronne (330) mobile en rotation et/ou être mobile en translation, la deuxième électrode ECG (170) étant formée par la couronne (330).

4. Dispositif (100) selon l'une quelconque des revendications précédentes, dans lequel le potentiel imposé est un potentiel constant.

5. Dispositif (100) selon l'une quelconque des revendications précédentes, dans lequel le module ECG (300) est configuré pour imposer le potentiel sur la deuxième électrode ECG (170).

6. Dispositif (100) selon l'une quelconque des revendications précédentes, dans lequel le module ECG (300) comprend un frontal analogique propre à recevoir une amplitude maximale de tension, le module ECG (300) étant configuré pour imposer un potentiel constant à une valeur environ égale à la moitié de l'amplitude maximale de tension.

7. Dispositif (100) selon la revendication 6, dans lequel le module ECG (300) est configuré pour imposer un potentiel à une valeur compris entre 0,5V et 2 V, notamment entre 0,8V et 1,0V.

8. Dispositif (100) selon l'une quelconque des revendications précédentes, dans lequel le fond de carrure (210) comprend un capteur optique (230), la première électrode (220) entourant au moins en partie le capteur optique (230).

9. Dispositif (100) selon la revendication 8, dans lequel la première électrode (220) entoure le capteur optique (230) sur une étendue angulaire supérieure à 180°.

10. Dispositif (100) selon la revendication 8 ou 9, dans lequel la première électrode (220) entoure entièrement le capteur optique (230).

11. Dispositif (100) selon l'une quelconque des revendications précédentes, dans lequel la première électrode (220) présente une forme annulaire.

12. Dispositif (100) selon l'une quelconque des revendications précédentes, dans lequel la première électrode (220) se présente sous la forme d'un corps métallique.

**13.** Dispositif (100) selon l'une quelconque des revendications précédentes, dans lequel la première électrode (220) se présente sous la forme d'un revêtement métallique.

**14.** Dispositif (100) selon l'une quelconque des revendications précédentes, dans lequel le dispositif électronique portatif (100) est une montre, la montre étant par exemple une montre hybride avec des aiguilles mécaniques.

**15.** Méthode de prise d'un électrocardiogramme, ECG, à l'aide d'un dispositif (100) selon l'une quelconque des revendications précédentes, durant laquelle l'utilisateur met un bras en contact avec la première électrode ECG (220) et un autre bras en contact avec la deuxième électrode ECG (170).

FIG. 1

FIG. 2

FIG. 3

100

430

440

410

420

450

300

390

395

460

470

FIG. 4

134

100

140, 170

520

510

530

540

550

FIG. 5

FIG. 6

FIG. 7

FIG. 8

FIG. 9

FIG. 10

(Etat de la technique)

FIG. 11

Europäisches
Patentamt

European
Patent Office

Office européen
des brevets

# RAPPORT DE RECHERCHE EUROPEENNE

Numéro de la demande

**EP 23 20 1697**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | US 2019/059756 A1 (RASMUSSEN ADAM B [US] ET AL) 28 février 2019 (2019-02-28)<br>* figures 1,6A,7,8 *<br>* alinéa [0026] *<br>* alinéa [0034] *<br>* alinéa [0045] – alinéa [0049] *<br>* alinéa [0057] – alinéa [0059] *<br>* alinéa [0060] – alinéa [0061] *<br>----- | 1-15 | INV.<br>A61B5/00<br>A61B5/0245<br>A61B5/308<br>A61B5/332<br>A61B5/024<br>A61B5/1455 |
| X | US 2019/072912 A1 (PANDYA SAMEER [US] ET AL) 7 mars 2019 (2019-03-07)<br>* figures 1B,2A,4A,23 *<br>* alinéa [0053] *<br>* alinéa [0066] – alinéa [0069] *<br>* alinéa [0085] – alinéa [0087] *<br>* alinéa [0265] – alinéa [0266] *<br>----- | 1,3,8,<br>11-15 | |
| A | US 2021/169420 A1 (JUNG HYUNJUN [KR])<br>10 juin 2021 (2021-06-10)<br>* alinéa [0138] – alinéa [0141] *<br>----- | 4-7 | |
| A | US 2017/075305 A1 (RYU GRAMI [KR] ET AL)<br>16 mars 2017 (2017-03-16)<br>* figure 10D *<br>* alinéa [0184] – alinéa [0185] *<br>----- | 14 | DOMAINES TECHNIQUES RECHERCHES (IPC)<br><br>A61B |

Le présent rapport a été établi pour toutes les revendications

| Lieu de la recherche | Date d'achèvement de la recherche | Examinateur |
|---|---|---|
| La Haye | 9 octobre 2023 | Oancea, A |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
..............................................................
& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 23 20 1697

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

09-10-2023

| Document brevet cité au rapport de recherche | Date de publication | Membre(s) de la famille de brevet(s) | Date de publication |
|---|---|---|---|
| US 2019059756 A1 | 28-02-2019 | AUCUN | |
| US 2019072912 A1 | 07-03-2019 | AU 2018329631 A1 | 16-01-2020 |
| | | AU 2021261944 A1 | 02-12-2021 |
| | | CN 111065984 A | 24-04-2020 |
| | | CN 209789844 U | 17-12-2019 |
| | | CN 213372029 U | 08-06-2021 |
| | | EP 3451117 A1 | 06-03-2019 |
| | | EP 4252632 A2 | 04-10-2023 |
| | | KR 20200027010 A | 11-03-2020 |
| | | KR 20220011803 A | 28-01-2022 |
| | | KR 20230087624 A | 16-06-2023 |
| | | TW 201912108 A | 01-04-2019 |
| | | TW 202029927 A | 16-08-2020 |
| | | US 2019072912 A1 | 07-03-2019 |
| | | US 2019101870 A1 | 04-04-2019 |
| | | US 2020229761 A1 | 23-07-2020 |
| | | US 2021204876 A1 | 08-07-2021 |
| | | US 2023098960 A1 | 30-03-2023 |
| | | US 2023210461 A1 | 06-07-2023 |
| | | WO 2019050778 A1 | 14-03-2019 |
| US 2021169420 A1 | 10-06-2021 | CN 114786580 A | 22-07-2022 |
| | | EP 4054419 A1 | 14-09-2022 |
| | | KR 20210073274 A | 18-06-2021 |
| | | US 2021169420 A1 | 10-06-2021 |
| | | WO 2021118095 A1 | 17-06-2021 |
| US 2017075305 A1 | 16-03-2017 | CN 106550106 A | 29-03-2017 |
| | | EP 3144792 A1 | 22-03-2017 |
| | | KR 20170033062 A | 24-03-2017 |
| | | US 2017075305 A1 | 16-03-2017 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5289824 A

- EP 2021058955 W

**Littérature non-brevet citée dans la description**

- **DE PINHO FERREIRA.** *A Review of Methods for Non-Invasive Heart Rate Measurement on Wrist,* 2020